Europäisches Patentamt

European Patent Office

Office européen des brevets

(11) Publication number: **0 033 548**
**B1**

(12) **EUROPEAN PATENT SPECIFICATION**

(45) Date of publication of patent specification: **14.05.86**

(21) Application number: **81100826.7**

(22) Date of filing: **05.02.81**

(51) Int. Cl.⁴: **A 61 B 17/10**

(54) **Surgical stapling instrument.**

(30) Priority: **05.02.80 US 118664**

(43) Date of publication of application:
**12.08.81 Bulletin 81/32**

(45) Publication of the grant of the patent:
**14.05.86 Bulletin 86/20**

(84) Designated Contracting States:
**AT BE CH FR IT LI NL SE**

(56) References cited:
**FR-A-2 310 117**
**US-A-3 499 591**

(73) Proprietor: **United States Surgical Corporation**
**150 Glover Avenue**
**Norwalk Connecticut 06850 (US)**

(72) Inventor: **Green, David Thomas**
**251 Wolfpit Avenue**
**Norwalk Connecticut 06851 (US)**

(74) Representative: **Lorenz, Eduard et al**
**Rechtsanwälte Eduard Lorenz - Bernhard Seidler**
**Margrit Seidler - Dipl.-Ing. Hans-K. Gossel Dr. Ina**
**Philipps - Dipl.-Chem. Rainer Wulf**
**Widenmayerstrasse 23**
**D-8000 München 22 (DE)**

EP 0 033 548 B1

Courier Press, Leamington Spa, England.

## Description

Technical Field

This invention relates to an instrument for use in applying surgical fasteners such as staples, clips and the like to living tissue, and to a staple cartridge for use with said instrument. More particularly, the invention relates to a surgical stapling instrument for use in forming a plurality of laterally spaced rows of staples in an internal body organ.

Typically, such an instrument comprises a pair of cooperating elongate jaw members, one of which in use carries a staple cartridge with at least two laterally spaced rows of staples and the other which carries an anvil with staple-closing depressions aligned with the rows of staples in the cartridge. A pusher bar and knife assembly is provided which is moved longitudinally along the jaws to sequentially eject staples from the cartridge by a camming action, through the agency of staple pushers carried by the cartridge in association with the individual staples, and to close the staples against the anvil thereby forming laterally spaced lines of staples in tissue gripped between the jaws, while the knife, which trails the pusher bars, cuts the tissue along a line between the staple rows. One instrument of this type is disclosed, for example, in U.S. Patent No. 3,499,591, the disclosure of which is incorporated herein by reference.

Background Art

With instruments of the above type, the staple cartridges and associated anvils, pusher bars and knife assemblies have commonly been made of disposable plastics and low cost metal stampings while the frames, namely the basic instruments have more generally been constructed for repeated usage necessitating continual sterilization of these elements between each use. In recent years, in order to obviate the need for such repeated sterilization of instruments, the tendency in the surgical field has been towards the introduction of fully self-contained disposable instruments which are used for only a single operation and then discarded. Obviously, therefore in the design of such disposable instruments, economics is a factor and it is desirable to design such instruments to utilize readily available economic materials of minimum material weight and to employ production techniques of optimum economy.

With stapling instruments of the type described, relatively large forces are involved in clamping the tissue to be fastened and in ejecting the individual staples, causing these to penetrate the gripped tissue and to be closed against the anvil. Such forces tend both to separate the jaws vertically and to laterally distort the jaws thereby hindering accurate stapling. This problem is of course accentuated if relatively light-weight disposable materials are to be used for manufacture of the jaw frames.

It is an object of the present invention to provide an instrument of the character described for applying surgical fasteners, in which optimum alignment and stabilization of the jaws is obtained during application and securing of the fasteners.

Another object of the invention is to provide a novel form of instrument for use in applying surgical fasteners to living tissue and which is particularly suited to use surgical stapling procedures.

It is a further object of the invention to provide a surgical stapling or like fastening instrument having a design which allows the instrument to be manufactured in the main from relatively light weight disposable materials while still providing proper alignment and stabilization of the jaws during stapling, and to provide an appropriate staple cartridge for use with said instrument.

Disclosure of Invention

In accordance with the invention, the elongate jaws of a surgical stapling instrument of the kind described are locally supported during stapling, substantially at the point where maximum jaw deflecting forces occur, by support means which move along the jaws with the pusher bar and knife assembly. To provide for lateral alignment and vertical stabilization of the jaws during stapling, each jaw is formed with a longitudinal channel or passageway and the pusher bar and knife assembly carries upper and lower support shoes which are accurately laterally aligned with one another and which fit closely in the respective passageway when the jaws are closed, so that the shoes travel along the passageways when the pusher bar and knife blade assembly is operated.

The shoes are located on the pusher bar and knife blade assembly in the region of the pusher bar cams and the knife blade and their effect is to provide localized support for the jaws in the region of these elements as they progress along the jaws, thereby providing proper jaw alignment in the region of the individual staples as these are sequentially acted upon by the pusher bars. Further, the shoes are not only laterally but also vertically confined in the passageways and thereby serve to resist vertical jaw-opening forces during staple formation.

Conveniently, the jaw passageways are formed towards the outside of the respective jaws and the shoes are carried on upper and lower extensions of the knife carrier which is made of relatively rigid material affording adequate lateral support to the shoes and hence to the jaws at the point of stapling as the shoes move therealong. To allow passage of the vertically extended knife carrier along the jaws, the staple cartridge and the anvil which are supported on the inner facing surfaces of the respective jaws are each composed of two laterally spaced elongate members carried by the respective jaws so that the extended knife carrier can move through spaces formed between the respective elongate members.

By utilizing the aforementioned shoes locally to support the jaws and provide both lateral and vertical stabilization in the region of the pusher bar cams and knife blade as these elements ride along the jaws, the adverse effects of the previously mentioned forces are substantially minimized and the jaws themselves can therefore be made of light-weight construction so that an instrument designed in accordance with the invention lends itself to manufacture in disposable materials.

Brief Description of Drawings

Figure 1 is a side view of a surgical stapling instrument in the assembled and closed condition;

Figure 2 is a side view of the instrument in an open condition;

Figure 3 is an exploded view of the instrument;

Figure 4 is a side view of a pusher bar and knife assembly;

Figure 5 is an underneath plan view of the pusher bar and knife assembly;

Figure 6 is a section on line 6—6 of Figure 1;

Figure 7 is a section on line 7—7 of Figure 1; with parts of the instrument removed;

Figure 8 is an underneath view of a disposable staple cartridge;

Figure 9 is a side view of the cartridge;

Figure 10 is a section on line 10—10 of Figure 9;

Figure 11 is a top view of an elongate anvil member;

Figure 12 is a side view of the anvil member;

Figure 13 is a section on line 13—13 of Figure 12; and

Figure 14 is a section on line 14—14 of Figure 1.

Best Mode for Carrying Out the Invention

It is to be noted that the general construction and principle of operation of the illustrated instrument is similar to the instrument described in U.S. Patent No. 3,499,591 previously referred to. Accordingly, the following description will only deal in detail with modified features of the instrument and for a fuller understanding of the principles and operation of the instrument, reference may be made to the above patent, the disclosure of which is incorporated herein by reference.

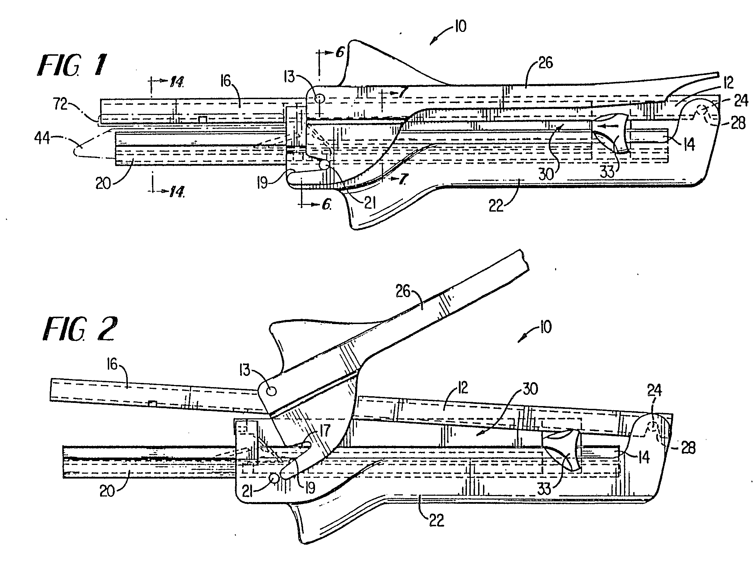

The present instrument, generally indicated by reference 10 includes an upper frame 12 and a lower frame 14. The forward end portion of upper frame 12 defines an elongate upper jaw 16 while the forward end portion of the lower frame 14 defines an elongate lower jaw 20. The portion of lower frame 14 rearward of jaw 20 fits into a channel-shaped handle member 22 having a pivot bar 24 at its rear end which is received in notches 28 at the rear end of upper frame member 12. Intermediate its length, upper frame member 12 has laterally projecting lugs 13 which fit in complimentary openings 15 formed in a bifurcated locking handle 26. The handle 26 can be used to open and close the frames about the pivot means 24, 28 between the open condition shown in Figure 2 and the locked condition shown in Figure 1. To this end, handle 26 has slots 17 providing camming surfaces 19 which cooperate with laterally projecting lugs 21 on handle member 22.

The instrument further includes a sliding pusher bar and knife assembly 30 comprising a central knife carrier 32 and laterally spaced pusher bars 34 and 36 on either side the knife carrier, the pusher bars terminating at their forward ends in inclined pusher bar cams 38 and 40, respectively, and the knife carrier including an inclined knife 42 situated just to the rear of the pusher bar cams.

In use, a disposable staple cartridge 44, containing four laterally spaced longitudinal rows of staples, is inserted into the lower jaw 20 while two anvil members 72 having staple shaping depressions in their outer surfaces, complimentary to the positioning of the individual staples in the staple cartridge, are placed on the upper jaw 16. The instrument is inserted into a patients body and manipulated such that tissue to be cut and sutured is inserted between the jaws, an incision to receive one of the jaws having previously been made in the tissue, if required. The jaws are then closed and locked by handle 26 to firmly grip the tissue between the opposing staple cartridge and anvil surfaces. The pusher bar and knife assembly which is initially in a rearward position relative to the jaws, is then pushed forwardly causing the pusher bar cams to enter longitudinal slits in the staple cartridge, in which slits are accommodated rows of individual staple pushers. The pusher bar cams cooperate sequentially with camming surfaces on the individual staple pushers to force the staples successively from the cartridge, through the gripped tissue and into engagement with the anvil depressions which thereby, in conjunction with the cams, produce staple-closing forces. The design of the cartridge is such that each slit carries two mutually staggered rows of staples so that in all, four staple rows are formed in the gripped tissue. The knife 42 which trails the pusher bars cams slightly and rides in central longitudinal slits in the cartridge and anvil, cuts the gripped tissue along a line between the two pairs of staple rows.

To provide lateral support for the jaws and to resist forces tending to vertically open the jaws during stapling, each jaw is provided with a longitudinal passageway and the knife blade carrier 32 which is a relatively rigid member, preferably of metal, carries upper and lower laterally aligned shoes which ride in these passageways and provide the required local support to the jaws in the region of the pusher bar cams and knife blade as these elements travel along the staple cartridge.

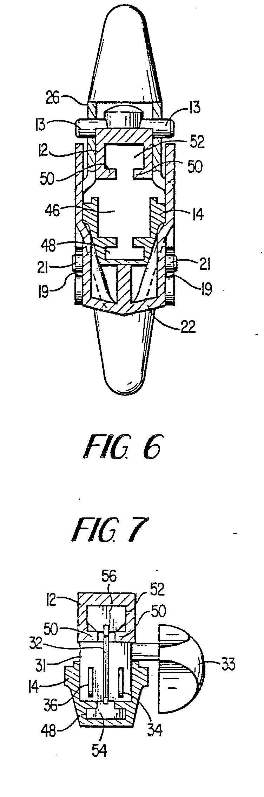

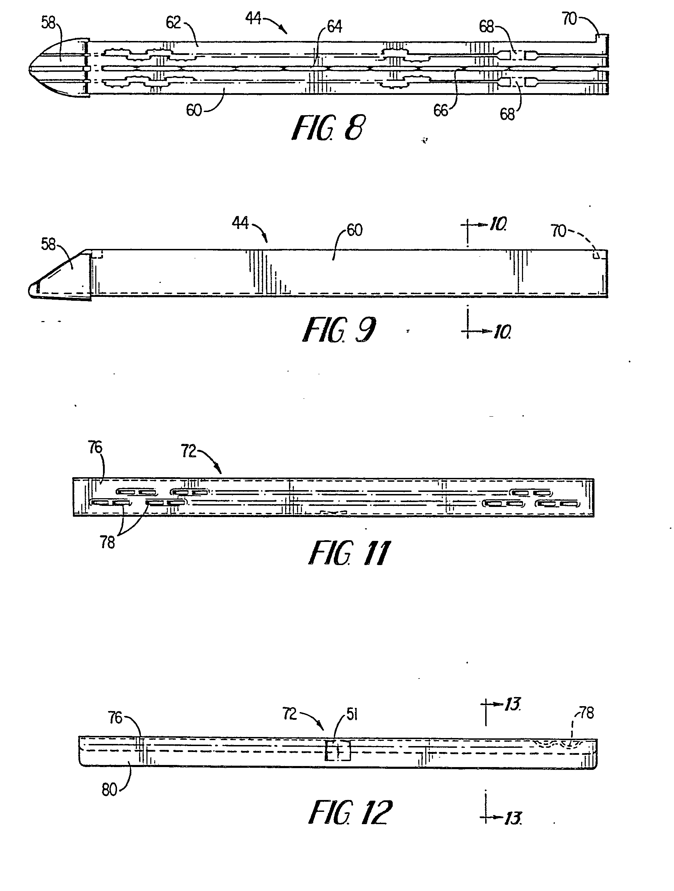

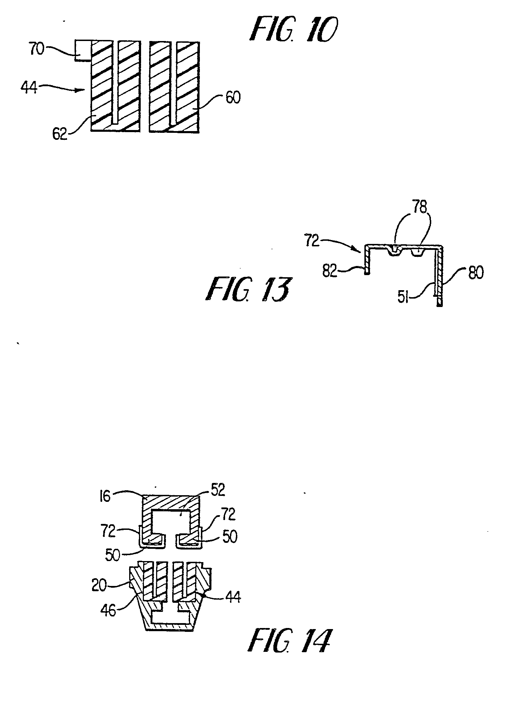

Thus, it will be seen, particularly in Figures 6, 7 and 14 that the lower frame 14 is formed with a cartridge-receiving channel 46 and an outer longitudinal passageway 48 of generally T-shaped cross-section extending from the base of the channel 46. Upper frame 12 has inwardly directed longitudinally extending shoulders 50 for mounting the anvils as will be described, the construction of the upper frame being such that a longitudinally extending passageway 52 of generally rectangular section is formed beneath the shoulders.

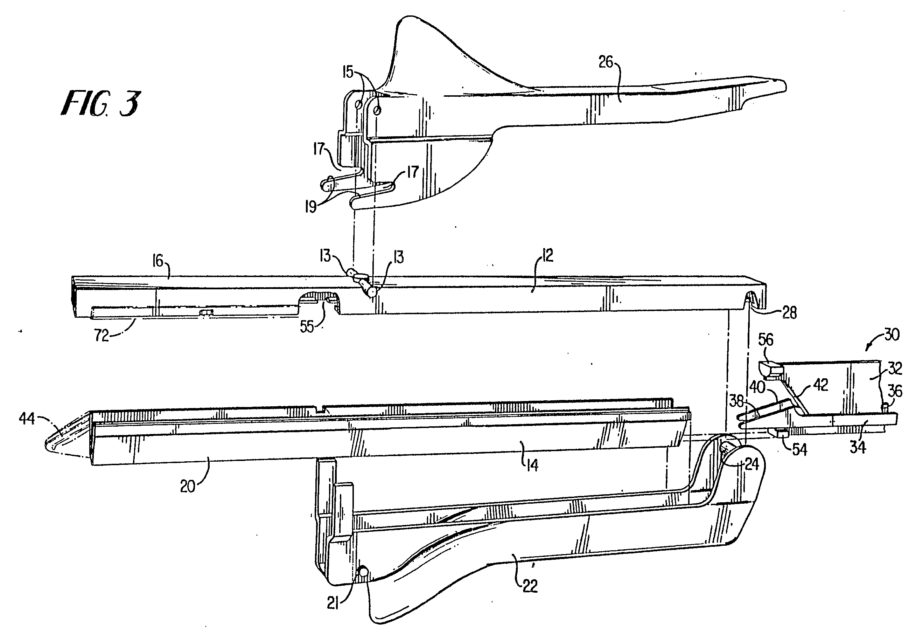

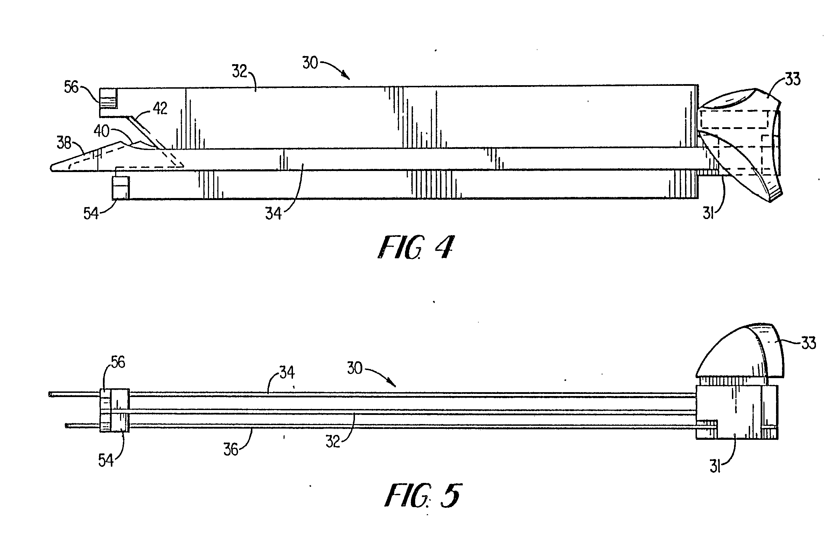

The pusher bar and knife assembly 30, see particularly Figures 3, 4 and 5, has conventional-type laterally spaced pusher bars 34 and 36 terminating in slightly offset inclined pusher bar cams 38 and 40. The central knife carrier 32, as indicated, has an inclined knife 42 just to the rear of the pusher bar cams and the knife carrier is extended vertically above and below the pusher bars. On upper and lower terminal portions of the knife carrier which project forwardly of the knife, are situated lower and upper shoes 54 and 56, respectively, the vertical spacing between the shoes corresponding to the vertical spacing between the passageways 48 and 52 in the lower and upper frames when the frames are locked together. The lower shoe 54 has a substantially T-shaped cross section corresponding to the cross-sectional shape of passageway 48 so that this shoe fits in passageway 48 with minimal clearance to allow substantially friction-free passage of the shoe along the passageway, and upper shoe 56 is likewise shaped to fit in passageway 52 with minimal clearance to provide substantially friction-free passage. It will be noted that the shoes 52 and 54 are longitudinally located in proximity to the pusher bar cams 38 and 40 so as to in use, provide support for the jaws 16 and 20 in the region of the cams substantially at points where the forces created by cooperation of the pusher bar cams and the individual staple pushers in the cartridge approach a maximum. Further, by locating the shoes both laterally and vertically in the respective passageways, both lateral alignment of the jaws and resistance to vertical jaw opening during stapling is obtained.

At the rear end of assembly 30, the pusher bars and knife carrier are mounted in known manner in a suitable carrying block 31 having an operating knob 33 or the like.

The staple cartridge 44 as shown in Figures 8, 9 and 10 is generally of similar character to the known type as described in the aforementioned U.S. Patent No. 3,499,591 insofar as the number of staple rows, and design and location of the individual staple pushers is concerned. For a fuller description of these elements and the manner in which staple ejection is effected by interaction of the pusher bar cams and the individual staple pushers, reference may therefore be made to this patent. In the present case, the cartridge 44 has a solid nose portion 58 and a pair of longitudinal ribs 60 and 62 extending rearwardly from the nose portion and defining a slit 64 therebetween right through the cartridge for passage of the knife carrier 32. The inner facing surfaces of the ribs 60 and 62 again have bumps 66 to laterally locate the knife carrier and the ribs are themselves longitudinally slit to accommodate the staple pushers and the opposed, staggered rows of staples. Friction pieces 68 are again provided to inhibit inadvertent forward motion of the pusher bars. Thus, the significant difference between the present cartridge and that described in U.S. Patent No. 3,499,591 is that in the present case slit 64 extends right through the cartridge (to the rear of the nose portion) to allow passage of the vertically extended knife carrier. In use, as indicated, the cartridge 44 sits in the cartridge receiving channel 46 of the upper jaw with the nose portion 58 projecting forwardly of the jaw and positive longitudinal location of the cartridge being effected by means of a projecting cartridge lug 70 and a complimentary cut-out in one of the side walls of channel 46.

While the instrument has been herein described as utilizing a separate staple cartridge 44 in like manner to the instrument described in U.S. Patent No. 3,499,591, it is contemplated within the scope of the present invention, for the staple cartridge to be formed as an integral part of lower jaw 20.

The anvil means in the present instance, see Figures 11, 12 and 13, comprises a pair of like individual elongate anvils 72 which sit with a friction fit on the shoulders 50 of the upper jaw, as seen in Figures 1, 3 and 14, whereby a slit is provided between the anvils allowing passage for the vertically extended knife carrier. Each individual anvil comprises an anvil surface 76 with staple-shaping depressions 78, an outer wall 80 and a shorter inner wall 82. The inner and outer walls are slightly inwardly tapered to provide the friction fit on shoulders 50 and the outer walls each have depressions 51 aligning with complimentary depressions in the side walls of the lower jaw for longitudinally locating the individual anvils and accurately aligning the staple shaping depressions with the individual staples in cartridge 44.

While the instrument as described includes separate anvils 72 which fit on the shoulders 50 of upper jaw 16, it is also possible, particularly in the manufacture of a low-cost disposable instrument, to dispense with the anvils themselves and form the staple shaping depressions, directly in the undersurfaces of shoulders 50 of the upper jaw.

Assembly of the various components of the instrument is effected in the following manner with particular reference to Figures 1 to 3. Initially, with a cartridge 44 correctly positioned on jaw 20 of lower frame 14, the forward end of pusher assembly 30 is inserted from the back into lower frame 14, with lower shoe 54 fitting in passageway 48, and assembly 30 is moved forwardly along frame 14 until the pusher bars enter the longitudinally slit ribs 60 and 62 of cartridge 44 and are arrested by the friction pieces 68. This position of pusher assembly 30 relative to frame 14 is shown in Figures 1 and 2. Frame 14 with the inserted pusher assembly 30 is then fitted into handle member 22, suitable complimentary locating means (not shown) being provided on the frame and handle member to longitudinally align these elements and prevent forward movement of frame 14 in member 22 during staple ejection.

Anvils 72 are fitted on jaw 16 and lugs 13 of frame 12 are fitted into openings 15 of handle 26. Then with handle 26 tilted upwardly, as shown in Figure 2, notches 28 are engaged with pivot bar 24 to complete the assembly of the instrument.

In use, the instrument in the assembled open condition, substantially as shown in Figure 2, is inserted into a body cavity, so that tissue to be stapled is accepted between jaws 16 and 20 and the instrument is then locked by manipulation of handle 26 and cooperation of camming surfaces 19 with lugs 21. It will be noted that openings 55 (see Figure 3) are provided in shoulders 50 of the upper frame 12 which allow shoes 56 to enter channel 52 as the instrument is closed. With tissue gripped between the jaws and the instrument in the condition as shown in Figure 1, stapling is effected in the manner described in the aforementioned patent by pushing forwarding on knob 33.

When stapling is completed, the pusher assembly 30 is retracted to the initial position, allowing shoes 56 to be removed from channel 52 through openings 55, so that the instrument can be opened.

It will be appreciated, since the shoes 54 and 56 are accurately laterally aligned and carried by a relatively rigid member, that during stapling, as the shoes move along the passageways 48 and 52 with minimal clearance, they provide adequate localized support to the jaws in the region of operation of the pusher bar cams and the particular individual staple pushers being actuated. Due to the cross-sectional shape of the shoes and passageways, such support resists forces tending both laterally to distort the jaws and to open the jaws vertically and accordingly the present construction lends itself to manufacturing the jaws in relatively light weight disposable materials. It is to be understood however that the construction can also be used in instruments manufactured from more conventional materials.

While only a single preferred embodiment of the invention has been described in detail, it is to be understood that the invention is not limited to its specific features and modifications are possible within the scope of the attached claims. Thus, while the invention has been particularly described in relation to its application in a surgical stapling instrument, the invention is not limited to this application. The invention may be applied to other fastening instruments having opposed jaws which require stabilization while fastening means are applied to living tissue gripped between the jaws. For example, the invention may be applied to instruments for applying certain types of surgical clips or instruments for applying surgical fastening devices of the type set out in U.S. Patent No. 4,060,089.

**Claims**

1. A surgical stapling instrument having first and second cooperating frames (12, 14) each provided with an elongate jaw (16, 20), one of said jaws being adapted to receive at least two laterally spaced longitudinal rows of staples, an elongate pusher bar and knife assembly slidable longitudinally relative to said jaws (16, 20) for sequentially ejecting staples from said one of said jaws and shaping the staples against anvil means (72) provided on the other of said jaws to form a pair of laterally spaced staple rows in tissue gripped between said jaws and for cutting the tissue along a line between said staple rows, said assembly including a pair of laterally spaced pusher bars (34, 36) each having a forward end portion including a pusher bar cam (38, 40) and a knife carrier (32) having a knife blade (42) located between said pusher bars (34, 36), characterized by cooperative support means (48, 52, 54, 56) associated with said pusher bar and knife assembly (30) and with said jaws (16, 20) respectively for locally supporting said jaws in the region of said pusher bar cams (38, 40) as said cams move along said jaws thereby resisting forces tending to deflect said jaws during ejection and shaping of the individual staples.

2. A surgical stapling instrument as defined in claim 1 wherein said support means includes means (48, 52, 54, 56) for resisting forces tending to laterally distort said jaws (16, 20).

3. A surgical stapling instrument as defined in claim 1 wherein said support means includes means (48, 52, 54, 56) for resisting forces tending to vertically separate said jaws.

4. A surgical stapling instrument as defined in claim 1 wherein said support means includes means (48, 52, 54, 56) for resisting forces tending to laterally distort said jaws and forces tending to vertically separate said jaws.

5. A surgical stapling instrument as defined in claim 1 wherein said support means includes means defining a longitudinal passageway (48, 52) in each of said jaws (16, 20) and upper and lower shoes (54, 56) carried by said pusher bar and knife assembly (30) in the region of said pusher bar cams (38, 40), said shoes (54, 56) fitting in said passageway (48, 52) and travelling therealong during longitudinal movement of said assembly (30) to provide said local support for said jaws (16, 20).

6. A surgical stapling instrument as defined in claim 5 wherein said passageway and shoes (54, 56) have complimentary cross-sectional shapes resisting localized lateral misalignment of the jaws (16, 20) and resisting vertical separation of the jaws.

7. A surgical stapling instrument as defined in claim 6 wherein at least one of said shoes (56, 54) and the corresponding jaw passageway (52, 48) have complimentary substantially T-shaped cross-sectional profiles.

8. A surgical stapling instrument as defined in claim 5 wherein said knife carrier (32) includes upper and lower portions projecting forwardly of said knife (42) and wherein said shoes (56, 54) are disposed on the projecting portions above and below the level of said pusher bar cams (38, 40) respectively.

9. A surgical stapling instrument as defined in claim 1 wherein said jaws (16, 20) are made of light-weight disposable plastics material and said knife carrier (32) is made of relatively rigid material whereby said shoes (56, 54) provide support to the jaws resisting said jaw deflecting forces.

10. A surgical stapling instrument as defined in claim 1 including a staple cartridge (44) for mounting on said one of said jaws (20) to provide said rows of staples, said cartridge comprising a pair of longitudinally extending staple-carrying elements (60, 62) defining a slit (64) therebetween for passage of said knife carrier (32) when said cartridge is mounted on one of said jaws (20).

11. A surgical stapling instrument as defined in claim 10 wherein said cartridge (44) has a common nose portion (58) and said staple-carrying elements (60, 62) each extend rearwardly from said nose portion.

12. A surgical stapling instrument as defined in claim 1 including anvil means comprising a pair of elongate anvil members (72) and means (50) for mounting said anvil members on the other said jaws (16) to define a slit therebetween for passage of said knife carrier (32).

13. A surgical stapling instrument as defined in claim 12 wherein each of said anvil members (72) is substantially channel-shaped in cross section and said other of said jaws (16) includes opposed inwardly directed longitudinal shoulders (50) defining said anvil mounting means, said anvil members seating on said shoulders with a friction fit.

14. A surgical stapling instrument as defined in claim 1 including means (24, 28) for pivotally connecting said frames (12, 14) together at the rear ends of said frames and a locking handle means (26) pivotally connected to one of said frames and cooperating with locking formations (21) associated with the other of said frames (14) for moving said jaws (16, 20) between open and closed conditions.

15. A surgical instrument for use in fastening living tissue comprising upper and lower cooperating elongated jaws (16, 20) movable between open and closed positions, whereby tissue to be fastened may be gripped between said jaws when said jaws are in the closed position, means (30) associated with one of said jaws (16) for mounting a tissue fastening means, a pusher means (34, 36) movable longitudinally relative to said jaws when said jaws are in said closed position for forceably expelling said fastening means from said one jaw and causing said fastening means to penetrate tissue gripped between the jaws support means carried by said pusher means, characterized in that said jaw support means (48, 52, 54, 56) cooperate with said jaws (16, 20) during movement of the pusher means (34, 36) along the jaws for resisting forces tending to separate said jaws during expulsion of said fastening means.

16. A surgical instrument as defined in claim 15 wherein said jaw support means (48, 52, 54, 56) includes means for resisting forces tending to laterally separate said jaws (16, 20) during expulsion of said fastening means.

17. A surgical instrument as defined in claim 15 or claim 16 wherein said support means includes means (48, 52, 54, 56) for resisting forces tending to vertically separate said jaws (16, 20) during expulsion of said fastening means.

18. A surgical instrument as defined in claim 15 wherein said support means includes upper and lower support shoes (56, 54) carried by said pusher means (34, 36) and wherein each of said jaws (16, 20) includes a longitudinal passageway (48, 50) for receiving the respective shoes, said shoes travelling in said passageway during movement of said pusher means (34, 36) along said jaws and locally supporting said jaws in the region of the respective shoes (56, 54).

19. A staple cartridge for use with a surgical stapling instrument, characterized by a solid nose portion (58), a pair of substantially parallel elongate limbs (60, 62) extending from said nose portion, said limbs defining an uninterrupted slit (64) therebetween extending the entire length and height of said limbs, seating means in each of said limbs for at least one longitudinally extending row of surgical staples and longitudinal passage means in each of said limbs (60, 62) for receiving a travelling pusher means (34, 36) operative sequentially to expel staples from said seating means.

20. A staple cartridge as defined in claim 19 including a series of individual staple pushers (34, 36) in each of said passage means, said staple pushers having camming surfaces (38, 40) for cooperating with said pusher means to expel staples from said seating means.

21. A staple cartridge as defined in claim 19 wherein said seating means in each of said limbs includes means of accommodating two mutually staggered rows of staples.

**Revendications**

1. Appareil pour la pose d'agrafes chirurgicales comportant des premier et second cadres coopérants (12, 14) pourvu chacun d'une mâchoire allongée (16, 20), une desdites mâchoires étant adaptée pour recevoir au moins deux rangées longitudinales d'agrafes espacées latéralement, un ensemble allongé de barre poussoir et de couteau se deplaçant dans le sens longitudinal par rapport auxdites mâchoires (16, 20) pour éjecter successivement des agrafes à partir de l'une desdites mâchoires et formant les agrafes contre un dispositif d'enclume (72) prévu sur l'autre desdites mâchoires pour former deux rangées d'agrafes espacées latéralement dans du tissu tenu entre lesdites mâchoires et pour couper le tissu le long d'une ligne entre lesdites rangées d'agrafes, ledit ensemble comprenant une paire de barres poussoires espacées latéralement (34, 36), chacune ayant une extrémité avant comportant une came de barre poussoir (38, 40) et un porteur de couteau (32) comportant une lame de couteau (42) logée entre lesdites barres poussoirs (34, 36), caractérisé en ce que les moyens de support coopérants (48, 52, 54, 56) associés audit ensemble (30) de barre poussoir et de couteau et auxdites mâchoires (16, 20) respectivement, pour étayer localement lesdites mâchoires dans la région desdites cames de barre poussoir (38, 40) au fur et à mesure que lesdites cames se déplacent le long desdites mâchoires en résistant ce faisant aux forces tendant à dévier lesdites mâchoires pendant l'éjection et la formation de chacune des agrafes.

2. Appareil pour la pose d'agrafes chirurgicales selon revendication 1 caractérisé en ce que lesdits moyens de support comportent des moyens (48, 52, 54, 56) pour résister aux forces tendant à déformer latéralement lesdites mâchoires (16, 20).

3. Appareil pour la pose d'agrafes chirurgicales selon revendication 1 caractérisé en ce que lesdits moyens de support comportent des moyens (48, 52, 54, 56) pour résister aux forces tendant à séparer les mâchoires verticalement.

4. Appareil pour la pose d'agrafes chirurgicales selon revendication 1 caractérisé en ce que lesdits moyens de support comportent des moyens (48, 52, 54, 56) pour résister aux forces tendant à déformer latéralement lesdites mâchoires et aux forces tendant à séparer verticalement lesdites mâchoires.

5. Appareil pour la pose d'agrafes chirurgicales selon revendication 1 caractérisé en ce que lesdits moyens de support comportent des moyens définissant un passage (48, 52) longitudinal dans chacune desdites mâchoires (16, 20) et des patins (54, 56) supérieur et inférieur portés par ledit ensemble (30) de barre poussoir et de couteau dans la région desdites cames de barres poussoirs (38, 40), lesdits patins (54, 56) étant adaptés audit passage (48, 52) et se déplaçant le long de ce dernier pendant le mouvement longitudinal dudit ensemble (30) pour fournir l'appui local auxdites mâchoires (16, 20).

6. Appareil pour la pose d'agrafes chirurgicales selon revendication 5 caractérisé en ce que lesdits passage et patins (54, 56) ont des formes complémentaires en coupe transversale qui résistent au désalignement latéral local des mâchoires (16, 20) et à la séparation verticale des mâchoires.

7. Appareil pour la pose d'agrafes chirurgicales selon revendication 6 caractérisé en ce qu'au moins un desdits patins (56, 54) et le passage (52, 48) de mâchoire correspondant ont des profils complémentaires dont la coupe transversale est substantiellement en forme de T.

8. Appareil pour la pose d'agrafes chirurgicales selon revendication 5 caractérisé en ce que le porteur de couteau (32) comprend des parties supérieure et inférieure formant une projection avant dudit couteau (42) et en ce que lesdits patins (56, 54) sont disposés sur les projections au-dessus et au-dessous respectivement desdites cames de barre poussoir (38, 40).

9. Appareil pour la pose d'agrafes chirurgicales selon revendication 1 caractérisé en ce que les mâchoires (16, 20) sont en matière plastique légère à jeter et que ledit porteur de couteau (32) est fabriqué dans une matière relativement rigide, lesdits patins (56, 54) fournissant un appui aux mâchoires en résistant auxdites forces de déviation des mâchoires.

10. Appareil pour la pose d'agrafes chirurgicales selon revendication 1 caractérisé en ce qu'une cartouche d'agrafes (44) destinée à être montée sur l'une desdites mâchoires (20) pour fournir lesdites rangées d'agrafes, ladite cartouche comportant une paire d'éléments (60, 62) s'étendant longitudinalement et portant les agrafes, et qui définissent une fente (64) à travers laquelle passe le porteur de couteau (32) lorsque la cartouche est montée sur l'une desdites mâchoires (20).

11. Appareil pour la pose d'agrafes chirurgicales selon revendication 10 caractérisé en ce que ladite cartouche (44) a un nez commun (58), et que lesdits éléments (60, 62) portant les agrafes sont disposés vers l'arrière à partir de ce nez.

12. Appareil pour la pose d'agrafes chirurgicales selon revendication 1 caractérisé en ce qu'une enclume est prévue comportant une paire d'éléments d'enclume (72) allongés et des moyens (50) pour monter lesdits éléments d'enclume sur l'autre desdites mâchoires (16) pour définir une fente dans laquelle passe ledit porteur de couteau (32).

13. Appareil pour la pose d'agrafes chirurgicales selon revendication 12 caractérisé en ce que chacun des éléments d'enclume (72) a une coupe transversale substantiellement en forme de U et que l'autre desdites mâchoires (16) comporte des épaulements (50) longitudinaux opposés dirigés vers l'intérieur définissant lesdits moyens de montage d'enclume, lesdits éléments d'enclume étant ajustés sur lesdits épaulements avec une certaine friction.

14. Appareil pour la pose d'agrafes chirurgicales selon revendication 1 caractérisé en ce que des moyens (24, 28) sont prévus pour raccorder entre eux par un joint pivotant lesdits cadres (12, 14) en leurs extrémités arrière et qu'une poignée de blocage (26) est raccordée par un joint pivotant à l'un desdits cadres et coopère avec un dispositif de blocage (21) associé avec l'autre desdits cadres (14) servant à déplacer lesdites mâchoires (16, 20) entre l'état ouvert et l'état fermé.

15. Appareil chirurgical destiné à être utilisé pour l'agrafage de tissus vivants comportant des mâchoires (16, 20) allongées, coopérantes, supérieure et inférieure pouvant se déplacer entre des positions ouvertes et fermées, le tissu qui doit être agrafé pouvant être tenu entre lesdites mâchoires lorsque lesdites mâchoires sont en position fermée, des moyens (30) associés à l'une desdites mâchoires (16) pour monter un dispositif d'agrafage de tissu, un dispositif poussoir (34, 36) pouvant être déplacé dans le sens longitudinal par rapport auxdites mâchoires lorsque lesdites mâchoires sont dans ladite position fermée pour éjecter par la force lesdits moyens d'agrafage à partir de l'une desdites mâchoires et amenant ledit moyen d'agrafage à pénétrer dans le tissu tenu entre les moyens d'appui des mâchoires que porte ledit dispositif poussoir, caractérisé en ce que lesdits moyens d'appui des mâchoires (48, 52, 54, 56) coopèrent avec lesdites mâchoires (16, 20) pendant le mouvement dudit dispositif poussoir (34, 36) le long des mâchoires pour résister aux forces tendant à séparer lesdites mâchoires pendant l'éxpulsion desdits moyens d'agrafage.

16. Appareil chirurgical selon revendication 15 caractérisé en ce que les moyens d'appui des mâchoires (48, 52, 54, 56) comportent des moyens pour résister aux forces tendant à séparer latéralement lesdites mâchoires (16, 20) pendant l'expulsion desdits moyens d'agrafage.

17. Instrument chirurgical selon revendication 15 ou 16 caractérisé en ce que lesdits moyens d'appui comportent des moyens (48, 52, 54, 56) pour résister aux forces tendant à séparer verticalement lesdites mâchoires (16, 20) pendant l'expulsion desdits moyens d'agrafage.

18. Instrument chirurgical selon revendication 15 caractérisé en ce que les moyens d'appui comportent des patins d'appui (56, 54) supérieur et inférieur portés par ledit dispositif poussoir (34, 36) et en ce que chacune desdites mâchoires (16, 20) comporte un passage longitudinal (48, 50) pour recevoir les patins correspondants, lesdits patins se déplaçant dans ledit passage pendant le mouvement dudit dispositif poussoir (34, 36) le long desdites mâchoires et servant localement d'appui auxdites mâchoires dans la région des patins correspondants (56, 54).

19. Cartouche d'agrafes à être utilisée avec un appareil pour la pose d'agrafes chirurgicales caractérisée en ce qu'un nez (58) solide est prévu ainsi qu'une paire de membres (60, 62) allongés essentiellement parallèles, s'étendant à partir dudit nez, lesdits membres définissant entre eux une fente ininterrompue (64) s'étendant sur toute la longueur et toute la hauteur desdits membres, qu'un logement est prévu dans chacun desdits membres pour recevoir au moins une rangée d'agrafes chirurgicales en sens longitudinal ainsi qu'un passage longitudinal dans chacun desdits membres (60, 62) pour recevoir un dispositif poussoir mobile (34, 36) à fonctionnement séquentiel pour expulser les agrafes dudit logement.

20. Cartouche d'agrafes selon revendication 19 comportant une série de poussoirs individuels d'agrafes (34, 36) dans chacun desdits passages, lesdits poussoirs d'agrafes ayant des surfaces à cames (38, 40) pour coopérer avec lesdits dispositifs poussoirs pour expulser les agrafes dudit logement.

21. Cartouche d'agrafes selon revendication 19 caractérisé en ce que ledit logement dans chacun desdits membres comporte des moyens pour recevoir deux rangées d'agrafes disposées en quinconce.

**Patentansprüche**

1. Instrument zum Anbringen von Wundklammern, mit einem ersten Rahmen (12) und einem damit zusammenwirkenden zweiten Rahmen (14), die jeweils mit einer langgestreckten Klemmbacke (16, 20) versehen sind, wobei die eine Klemmbacke mindestens zwei mit seitlichem Abstand voneinander verlaufende Längsreihen von Wundklammern aufnehmen kann, ferner eine langgestreckte Anordnung aus Vorschubstange und Messer, die in Längsrichtung gegenüber den Klemmbacken (16, 20) verschiebbar ist, um nacheinander Wundklammern aus der einen Klemmbacke auszugeben und die Wundklammern an einem Amboß (72) zu formen, der an der anderen Klemmbacke vorgesehen ist, um zwei mit seitlichem Abstand voneinander verlaufende Reihen von Wundklammern in dem zwischen den Klemmbacken gegriffenen Gewebe zu bilden und um das Gewebe längs einer Linie zwischen den Wundklammerreihen zu schneiden, wobei die Anordnung zwei mit seitlichem Abstand voneinander verlaufende Vorschubstangen (34, 36), an deren vorderem Ende jeweils eine Vorschubstangenschulter (38, 40) vorgesehen ist, sowie einen Messerträger (32) mit einer Messerklinge (42) zwischen den Schubstangen (34, 36) aufweist, gekennzeichnet durch eine zusammenwirkende Abstützeinrichtung (48, 52, 54, 56) in Zuordnung zu der Anordnung (30) aus Vorschubstange und Messer bzw. zu den Klemmbacken (16, 20) zum örtlichen Abstützen der Klemmbacken im Bereich der Vorschubstangenschultern (38, 40), wenn die Schultern sich längs der Klemmbacken bewegen, wodurch den Kräften, die die Klemmbacken während des Ausgebens und Formens der einzelnen Wundklammern abzubiegen suchen, Widerstand entgegengesetzt wird.

2. Instrument zum Anbringen von Wundklammern, nach Anspruch 1, dadurch gekennzeichnet, daß die Abstützeinrichtung Elemente (48, 52, 54, 56) aufweist, durch welche Kräften, die die Klemmbacken (16, 20) abzubiegen suchen, Widerstand entgegengesetzt wird.

3. Instrument zum Anbringen von Wundklammern, nach Anspruch 1, dadurch gekennzeichnet, daß die Abstützeinrichtung Elemente (48, 52, 54, 56) aufweist, durch welche Kräften, die die Klemmbacken in vertikaler Richtung zu trennen suchen, Widerstand entgegengesetzt wird.

4. Instrument zum Anbringen von Wundklammern, nach Anspruch 1, dadurch gekennzeichnet, daß die Abstützeinrichtung Elemente (48, 52, 54, 56) aufweist, durch welche Kräften, die die Klemmbacken seitlich abzubiegen suchen, und Kräften, die die Klemmbacken in vertikaler Richtung zu trennen suchen, Widerstand entgegengesetzt wird.

5. Instrument zum Anbringen von Wundklammern, nach Anspruch 1, dadurch gekennzeichnet, daß die Abstützeinrichtung Elemente aufweist, die einen längsverlaufenden Durchlaß (48, 52) in jeder der beiden Klemmbacken (16, 20) bilden sowie einen unteren und einen oberen Gleitkörper (54, 56), die von der Anordnung (30) von Vorschubstangen und Messer im Bereich der Vorschubstangenschultern (38, 40) getragen werden, wobei die Gleitkörper (54, 56) dem Durchlaß (48, 52) angepaßt sind und sich in diesem bei der Längsbewegung der Anordnung (30) verlagern, um die örtliche Abstützung für die Klemmbacken (16, 20) zu bieten.

6. Instrument zum Anbringen von Wundklammern, nach Anspruch 5, dadurch gekennzeichnet, daß Durchlaß und Gleitkörper (54, 56) komplementär geformte Querschnittsflächen aufweisen, die einer örtlichen seitlichen Versetzung der Klemmbacken (16, 20) und der vertikalen Trennung der Klemmbacken Widerstand entgegensetzen.

7. Instrument zum Anbringen von Wundklammern, nach Anspruch 6, dadurch gekennzeichnet, daß mindestens einer der Gleitkörper (56, 54) und der zugeordnete Klemmbackendurchlaß (52, 48) komplementäre, im wesentlichen T-förmige Querschnittsflächen aufweisen.

8. Instrument zum Anbringen von Wundklammern, nach Anspruch 5, dadurch gekennzeichnet, daß der Messerträger (32) einen oberen und einen unteren Abschnitt aufweist, die vor das Messer (42) vorspringen, und daß die Gleitkörper (56, 54) auf den vorspringenden Abschnitten oberhalb bzw. unterhalb der Höhe der Schubstangenschultern (38, 40) angeordnet sind.

9. Instrument zum Anbringen von Wundklammern, nach Anspruch 1, dadurch gekennzeichnet, daß die Klemmbacken (16, 20) aus leichtem Kunststoff zum Einmalgebrauch hergestellt sind und der Messerträger (32) aus relativ festem Material besteht, wodurch die Gleitkörper (56, 54) eine Abstützung für die Klemmbacken bieten und den die Klemmbacken abbiegenden Kräften Widerstand entgegensetzen.

10. Instrument zum Anbringen von Wundklammern, nach Anspruch 1, gekennzeichnet durch eine Klammerkassette (44) zum Anbringen an der einen Klemmbacke (20) zur Bildung der Wundklammerreihen, wobei die Kassette zwei längsverlaufende Klammerntragelemente (60, 62) aufweist, zwischen denen ein Schlitz (64) ausgespart ist, durch den der Messerträger (32) hindurchtritt, wenn die Kassette in die eine Klemmbacke (20) eingesetzt wird.

11. Instrument zum Anbringen von Wundklammern, nach Anspruch 10, dadurch gekennzeichnet, daß die Kassette (44) einen gemeinsamen Nasenteil (58) besitzt, und daß die Klammerntragelemente (60, 62) jeweils von diesem Nasenteil aus nach hinten verlaufen.

12. Instrument zum Anbringen von Wundklammern, nach Anspruch 1, gekennzeichnet durch eine Amboßanordnung mit zwei langgestreckten Amboßteilen (72) und einer Einrichtung (59) zum Anbringen der Amboßteile an der anderen Klemmbacke (16), so daß dazwischen ein Schlitz für den Durchtritt des Messerträgers (32) verbleibt.

13. Instrument zum Anbringen von Wundklammern, nach Anspruch 12, dadurch gekennzeichnet, daß jedes Amboßteil (72) im wesentlichen den Querschnitt eines U-Profils hat, und daß die andere Klemmbacke (16) einander gegenüberstehende, nach innen gerichtete Längsschultern (50) aufweist, die die Halterung für den Amboß darstellen, wobei die Amboßteile mit Reibungspassung auf den Schultern sitzen.

14. Instrument zum Anbringen von Wundklammern, nach Anspruch 1, gekennzeichnet durch eine Einrichtung (24, 28) zum schwenkbaren Verbinden der Rahmen (12, 14) miteinander an den hinteren Enden der Rahmen, und durch einen Verriegelungsgriff (26), der mit dem einen Rahmen schwenkbar verbunden ist und mit einer Verschließeinrichtung (21) zusammenwirkt, die an dem anderen Rahmen (14) angebracht ist, um die Klemmbacken (16, 20) aus einer Offenstellung in eine Schließstellung und umgekehrt zu führen.

15. Chirurgisches Instrument zum Befestigen von lebendem Gewebe, mit einer oberen bzw. einer unteren langgestreckten Klemmbacke (16 bzw. 20), die zusammenwirken und aus einer offenen in eine geschlossene Stellung und umgekehrt bringbar sind, wodurch das zu befestigende Gewebe zwischen diesen Klemmbacken gegriffen werden kann, wenn die Klemmbacken sich in der geschlossenen Stellung befinden, mit einer mit der einen Klemmbacke (16) verbundenen Einrichtung (30) zum Anbringen einer Einrichtung zum Befestigen des Gewebes, einer Vorschubeinrichtung (34, 36), die gegenüber den Klemmbacken in Längsrichtung bewegbar ist, wenn die Klemmbacken geschlossen sind, um die Befestigungsmittel aus der einen Klemmbacke auszustoßen und das Befestigungsmittel zu veranlassen, das zwischen der von der Vorschubeinrichtung getragenen Klemmbackenabstützung gegriffene Gewebe zu durchdringen, dadurch gekennzeichnet, daß die Klemmbackenabstützeinrichtung (48, 52, 54, 56) mit den Klemmbacken (16, 20) während der Bewegung der Vorschubeinrichtung (34, 36) längs der Klemmbacken zusammenwirkt, um Kräften, die die Klemmbacken während des Ausstoßens des Befestigungsmittels voneinander zu trennen suchen, Widerstand entgegenzusetzen.

16. Chirurgisches Instrument nach Anspruch 15, dadurch gekennzeichnet, daß die Klemmbackenabstützeinrichtung (48, 52, 54, 56) eine Einrichtung aufweist, welche Kräften, die die Klemmbacken (16, 20) während des Ausstoßens des Befestigungsmittels seitwärts voneinander zu trennen suchen, Widerstand entgegensetzt.

17. Chirurgisches Instrument nach Anspruch 15 oder 16, dadurch gekennzeichnet, daß die Abstützeinrichtung eine Einrichtung (48, 52, 54, 56) aufweist, welche Kräften, die die Klemmbacken (16, 20) während des Ausstoßens des Befestigungsmittels in vertikaler Richtung zu trennen suchen, Widerstand entgegensetzt.

18. Chirurgisches Instrument nach Anspruch 15, dadurch gekennzeichnet, daß die Abstützeinrichtung von der Vorschubeinrichtung (34, 36) gehaltene obere und untere Gleitkörper (56, 54) aufweist, und daß in jeder Klemmbacke (16, 20) ein längsverlaufender Durchlaß (48, 50) für die Aufnahme des zugeordneten Gleitkörpers vorgesehen ist, wobei die Gleitkörper sich während der Bewegung der Vorschubeinrichtung (34, 36) längs der Klemmbacken in dem Durchlaß verschieben und die Klemmbacken in dem Bereich des zugeordneten Gleitkörpers (56, 54) örtlich abstützen.

19. Klammerkassette zur Verwendung bei einem Instrument zum Anbringen von Wundklammern, gekennzeichnet durch einen festen Nasenabschnitt (58), ein Paar im wesentlichen paralleler, langgestreckter, von dem Nasenabschnitt ausgehender Glieder (60, 62), die zwischen sich einen fortlaufenden Schlitz (64) bilden, der über die gesamte Länge und Höhe dieser Glieder reicht, durch eine Aufnahmeeinrichtung in beiden Gliedern für mindestens eine längsverlaufende Reihe von Wundklammern und durch einen längsverlaufenden Durchlaß in jedem Glied (60, 62) für die Aufnahme einer verschiebbaren Vorschubeinrichtung (34, 36), die nacheinander Wundklammern aus der Aufnahmeeinrichtung ausgibt.

20. Klammerkassette nach Anspruch 19, gekennzeichnet durch eine Reihe von einzelnen Klammernschiebern (34, 36) in jedem Durchlaß, wobei die Klammernschieber Schulterflächen (38, 40) zum Zusammenwirken mit der Vorschubeinrichtung besitzen, um Wundklammern aus der Aufnahmeeinrichtung auszugeben.

21. Klammerkassette nach Anspruch 19, dadurch gekennzeichnet, daß die Aufnahmeeinrichtung in jedem der Glieder eine Einrichtung für die Aufnahme von zwei gegeneinander versetzten Reihen von Klammern aufweist.

FIG. 1

FIG. 2

FIG. 3

0 033 548

FIG. 4

FIG. 5

FIG. 6

FIG. 7

*FIG. 8*

*FIG. 9*

*FIG. 11*

*FIG. 12*

*FIG. 10*

*FIG. 13*

*FIG. 14*